(19) Europäisches Patentamt
European Patent Office
Office européen des brevets

(11) **EP 2 293 687 B1**

(12) **EUROPEAN PATENT SPECIFICATION**

(45) Date of publication and mention
of the grant of the patent:
**28.10.2015 Bulletin 2015/44**

(21) Application number: **09735856.8**

(22) Date of filing: **24.04.2009**

(51) Int Cl.:
*A23K 1/16* (2006.01)     *A23K 1/18* (2006.01)

(86) International application number:
**PCT/NO2009/000156**

(87) International publication number:
**WO 2009/131467 (29.10.2009 Gazette 2009/44)**

(54) **FUNCTIONAL FEED COMPOSITION**

FUNKTIONELLE FUTTERMITTELZUSAMMENSETZUNG

COMPOSITION D ALIMENT POUR ANIMAUX FONCTIONNELLE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **24.04.2008  NO 20081977**

(43) Date of publication of application:
**16.03.2011  Bulletin 2011/11**

(73) Proprietors:
• **Ewos Innovation AS**
**4335 Dirdal (NO)**
• **Chemoforma Ltd**
**4302 Augst (CH)**

(72) Inventors:
• **GONZÁLEZ VECINO, José Luis**
**4326 Sandnes (NO)**
• **WADSWORTH, Simon**
**9415 Harstad (NO)**

(74) Representative: **Acapo AS**
**P.O. Box 1880 Nordnes**
**5817 Bergen (NO)**

(56) References cited:
**EP-A1- 1 082 908       WO-A1-2008/051091
WO-A2-2007/095313**

• LI P ET AL: "Nucleotide nutrition in fish: Current knowledge and future applications" AQUACULTURE, ELSEVIER, vol. 251, no. 2-4, 28 February 2006 (2006-02-28), pages 141-152, XP025040761 ISSN: 0044-8486 [retrieved on 2006-02-28]
• BURRELLS C ET AL: "Dietary nucleotides: A novel supplement in fish feeds: 1. Effects on resistance to disease in salmonids" 16 July 2001 (2001-07-16), AQUACULTURE, VOL. 199, NR. 1-2, PAGE(S) 159-169 , XP002536762 ISSN: 0044-8486 the whole document
• ZHOU J ET AL: "Effects of dietary supplementation of A3alpha-peptidoglycan on innate immune responses and defense activity of Japanese flounder (Paralichthys olivaceus)" AQUACULTURE, ELSEVIER, vol. 251, no. 2-4, 28 February 2006 (2006-02-28), pages 172-181, XP025040765 ISSN: 0044-8486 [retrieved on 2006-02-28]

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a feed composition, which can be administered to prevent or reduce the symptoms of infectious diseases in animals, the use of the feed composition and a method for feeding of fish as defined in the preamble of the independent claims.

BACKGROUND OF THE INVENTION

*Piscirickettsia salmonis* is a small, intracellular bacterium that causes a fatal septicaemia in salmonids. Since the initial isolation in the late 1980's, *P. salmonis* has been the primary cause of mortality of salmonids in the aquaculture industry in Chile. Because *P. salmonis* is intracellular, efficiency of antibiotic treatment of infected salmon is poor. Vaccine development has also proved difficult due to the intracellular nature of the bacterium. Current vaccines against *P. salmonis* are therefore largely ineffective after 6 months after the transfer from freshwater to seawater and the industry is still depending to a large degree on antibiotics, when treating this disease. Use of antimicrobial agents, such as oxolinic acid, has been extensive for a number of years in Chile, reaching over 130 t (active compound) per year. Over 80% of this volume is used to control *P. salmonis,* although no antimicrobial has proven to be consistently effective.

Moreover, antibiotic treatments such as with oxytetracycline and oxolinic acid can significantly reduce the functions of the specific and non-specific immune system of fish such as the Atlantic salmon. This immune suppression increases the risk of re-infection by pathological organisms such as by *P. salmonis,* leading to a rapid re-colonization of *P. salmonis* and repeated requirement of antibiotic administration. This extensive re-use of antibiotics further reduces the immunity of the fish and affects the natural microbial flora of the organisms such as the gut microflora. Lower feeding efficiency and growth are some of the reported negative effects of antibiotic treatments in this context and are thus not only an economical issue but also an issue of general fish welfare. Increasing public and consumer awareness of fish welfare increases the need for effective alternative treatments and prevention of disease outbreaks in aquaculture production.

The continued use of antibiotics has also raised residue and food safety concerns. It is now a requirement for all batches of harvested salmon from Chile for both the US and Japanese markets to be tested for antibiotic residues. These initiatives have resulted in a longer withdrawal period of over 2 months for Atlantic salmon populations prior to harvest. This period therefore represents a significant risk and can lead to high losses of valuable fish in case of serious infections of all kind. Moreover, any infection and losses in this pre-harvest population can result in significant financial penalty as no antibiotics can be used to control the disease.

Immune stimulants enhancing the immune response of fish have an important application in aquaculture, especially when deployed as part of an integrated health management program. Peptidoglycans (PGs) are structural components of bacterial cell walls and can form up to 90% dry weight of gram-positive bacteria. PGs are responsible for cell strength, shape and counteracting the osmotic pressure of the cytoplasm. They are formed from two alternating amino sugars that produce a strong lattice type structure. Due to their presence in many pathogenic bacteria PGs produce a strong response when exposed to a host's immune system. PGs have demonstrated improved protection against pathogens in a range of juvenile as well as adult aquaculture species including salmonids, yellowtail, tilapia, flounder and shrimp. However, it has been shown that the administration of peptidoglycan can result in negative side effects such as reduced survival, if it is not administered in an optimal dose, such as being too high or too low, and especially if administered for longer periods to the organism. From an aquaculture point of view, it would be beneficial if this strong immune stimulant can be administered for a longer period than what is recommendable based upon today's experimental experiences and knowledge. Matsuo K. & Miyazono I. ("The influence of long-term administration of peptidoglycan on disease resistance and growth of juvenile Rainbow-trout. Nippon Suisan Gakkaishi 89 (8): 1377-1379, August 1993) reported that oral administration of PG longer administration than 28 days can lead to decrease in disease resistance.

Zhou et al. (Effects of dietary supplementation of A3$\alpha$-peptidoglycan on innate immune responses and defense Japanese flounder (Paralichthys olivaceus). 2006. Aquaculture 251, pp. 172-181) describes the effect of dietary supplementation of A3$\alpha$-peptidoglycan on the immune response of Japanese flounder, also considering dose and administration length.

EP1082908A1 discloses a feedstuff additive for crustaceans and fishes comprising a low molecular weight lipopolysaccharide derived from gram-negative bacteria being capable of activating the immunity and preventing infections in the animals.

Most cells produce nucleotides for use in cell replication. Under normal conditions in any living organism the constant synthesis of nucleotides is well balanced with the natural demands needed for cell proliferation.

Burrells et al. (Dietary nucleotides: a novel supplement in fish feeds. Effects on resistance to disease in salmonids. 2001. Aquaculture 199, pp 159-169) and Li et al. (Nucleotide nutrition in fish: Current knowledge and future applications. 2006. Aquaculture 251, pp 141-152) describe the effects of inclusion of exogenous nucleotides in aquaculture diets on the resistance of fish to infections.

WO 2007/095313 A2 discloses a feed supplement comprising nucleotides having an improved water stability and dietary efficacy due to microencapsulation. The microcapsules may also comprise an immune stimulant such as a peptidoglycan.

WO 2008/051091 A1 (published May 2, 2008), a prior right according to Art. 54(3) in respect to novelty, discloses a feed comprising manna-oligosaccharides, optionally combined with nucleotides and an immune stimulant as peptidoglycan to compensate for negative effects of soy bean meal in diets.

The problem to be solved by the present invention is to develop and provide an effective functional health diet and feeding regime without the above mentioned negative side effects and which improves survival of fish during periods when the fish is or has been exposed to infections such as by *P. salmonis.* Such a diet would result in considerable economical benefits to both fish and feed producers. Moreover, it will contribute to improve fish well fare and can reduce the need and amount of antibiotic administration with all the known disadvantages.

## SUMMARY OF THE INVENTION

According to a first aspect of the invention, there is provided a fish feed composition comprising conventional feed ingredients characterized in that the composition further comprises peptidoglycan and nucleotides, wherein said peptidoglucan in the feed composition is in the range of 0.001-0.01%, more preferably 0.001-0.005 % and most preferably 0.001 % by weight.

Preferably, the nucleotides are in the range of 0.05 % -1 % by weight, more preferably 0.1-0.5 % by weight and most preferably of 0.2 % by weight.

Preferable, the weight ratio of nucleotides to peptidoglucan is more than 4, more preferable more than 20, and more preferable more than 40, and the amount of peptidoglucan is in the range of 0.001-0.01 % of the weight of the feed composition.

The nucleotides are chosen from adenosine monophosphate, cytidine monophosphate, guanosine monophosphate, uridine monophosphate, thymidine monophosphate.

Preferable, the fish feed composition above is a functional feed composition for preventing or reducing the symptoms related of an infectious disease in a fish.

Preferable, the fish feed composition above is for increasing the survival and/or growth of fish challenged by an infection.

Preferable, the infection is caused by bacteria such as *Piscirikettsia salmonis, Moritella viscosa, Francisella* sp, Mouth Rot, Streptococcal infections, *Vibrio* infections; Pancreas disease, NSAV, gill inflammation (GI), heart and skeletal muscle inflammation (HSMI), infectious salmonid anaemia (ISA) virus, Saprolegniosis, and sea lice infestation.

Preferable, the fish is a salmonid, preferable an Atlantic salmon.

Preferable, the fish feed composition is for effective recovery following an infection and/or antibiotic treatment due to an infection and/or to reduce the risk of a re-infection.

According to a second aspect, the invention comprises the use of a composition as indicated above for feeding of fish which are susceptible to an infection wherein the composition is provided to the fish in the period previous of the challenge by an infection, wherein the composition is fed for a period of 1-12 weeks prior to the infection.

Preferable, the composition is fed for a period of 2-6 weeks prior to infection, and most preferably of 4 weeks prior to infection.

Preferable, the fish is a salmonid, preferable an Atlantic salmon.

## DESCRIPTION OF THE INVENTION

Embodiments of the invention will now be described, by the way of examples with reference to the following diagrams, wherein:

Figure 1 shows the cumulative mortality (mean $\pm$ SEM) in experiment 1 during the post-challenge period of Atlantic Salmon (*Salmo salar*) fed different diets in the pre-challenge phase.

Figure 2 shows the Kaplan-Meier survival curves (Relative Percentage of Survival, mean $\pm$ standard error of the mean) of Atlantic salmon (*Salmo salar*) during the post-challenge period (experiment 1) after being fed different diets in the pre-challenge phase.

Figure 3 shows the cumulative mortality (%) (mean $\pm$ SDEV) of Atlantic salmon (*Salmo salar*) in experiment 2 expressed as time series. Fish were fed different diets prior to the *Piscirickettsia salmonis* challenge.

Figure 4 shows the cumulative mortality (%) (mean $\pm$ SEM) of Atlantic Salmon (*Salmo salar*) in experiment 2 expressed as final mortality after the post-challenge phase. Fish were fed different diets prior to the challenge with *Piscirickettsia salmonis.*

EXPERIMENTAL SECTION

Experiment 1

A total of 640 Atlantic salmon *Salmo salar* (160 fish per group), mixed sex, start weight 80g, seawater adapted, were fed for a period for 4 weeks at 2% body weight per day. In practice, the fish has free access to feed. Fish were maintained in 6 tanks. The fish were pit-tagged to identify the different groups, before the start of the feeding period. Fish were maintained in ambient seawater temperature (16°C). Mortalities were assessed on a daily basis.

There were 4 different dietary treatments in the pre-challenge period (Table 3) including 5 different diets (Table 1). Three groups of fish were fed a diet comprising nucleotides for 3 weeks followed by three different diets comprising peptidoglycan in different concentrations and nucleotides for one week. The control group received a commercial diet during the whole pre-challenge period of 4 weeks. All diets comprised conventional feed ingredients and did only differ in their amount of added peptidoglycan and nucleotides (Table 2).

Challenge with *Piscirikettsia salmonis* and post-challenge feeding

At 24 hours after the final feeding in the pre-challenge period the fish were mixed equally into 6 tanks with 25 fish per group per tank (100 fish total per tank). Fish were challenged with 0.2 ml of a lethal dose ($LD_{50}$) of *Piscirikettsia salmonis* by intra-peritoneal injection. After the challenge fish were fed the same control diet without any added nucleotides or peptidoglycan for 30 days (post-challenge) when the experiment was terminated and the final mortalities assessed.

Table 1: Test diets used in experiment 1. PG = peptidoglycan.

| Diet No. | Diet name |
|---|---|
| 1. | Control diet (Commercial) |
| 2. | Diet with 0.2 % nucleotides and PG 0.005%; (50g PG tonne$^{-1}$) |
| 3. | Diet with 0.2 % nucleotides and PG 0.01%; (100g PG tonne$^{-1}$) |
| 4. | Diet with 0.2 % nucleotides and PG 0.05%; (500g PG tonne$^{-1}$) |
| 5. | Diet with 0.2 % nucleotides |

Table 2: Main feed ingredients and chemical analysis of the composition of the test diets in experiment 1.

| Ingredient (% by weight)) | Diet 1 | Diet 2 | Diet 3 | Diet 4 | Diet 5 |
|---|---|---|---|---|---|
| Fish meal | 51 | 51 | 51 | 51 | 51 |
| Fish oil | 18 | 18 | 18 | 18 | 18 |
| Wheat gluten | 7 | 7 | 7 | 7 | 7 |
| Functional feed composition | 8 | 8 | 8 | 8 | 8 |
| Vitamin premix* | 0.2 | 0.2 | 0.2 | 0.2 | 0.2 |
| Mineral premix** | 0.3 | 0.3 | 0.3 | 0.3 | 0.3 |
| Peptidoglycan | - | 0.005 | 0.01 | 0.05 | - |
| Nucleotides | - | 0.2 | 0.2 | 0.2 | 0.2 |
| **Composition** (%) | | | | | |
| Protein | 47.9 | 47.9 | 47.9 | 47.9 | 47.9 |
| Lipid | 24.3 | 24.3 | 24.3 | 24.3 | 24.3 |
| Ash | 7.5 | 7.5 | 7.5 | 7.5 | 7.5 |
| Dry mass | 92.7 | 92.7 | 92.7 | 92.7 | 92.7 |

*EWOS Vitamin premix ®
**EWOS Mineral premix ®.

Table 3. Test diet feeding regimes in experiment 1 prior to the challenge with *Piscirikettsia salmonis* (pre-challenge) and after the challenge with *P. salmonis* (post-challenge).

| Treatment | Pre-challenge | | | | Post-challenge 30 days |
|---|---|---|---|---|---|
| | Week 1 | Week 2 | Week 3 | Week 4 | |
| 1 (control) | Diet 1 | Diet 1 | Diet 1 | Diet 1 | |
| 2 | Diet 5 | Diet 5 | Diet 5 | Diet 2 | Diet 1 |
| 3 | Diet 5 | Diet 5 | Diet 5 | Diet 3 | |
| 4 | Diet 5 | Diet 5 | Diet 5 | Diet 4 | |

LD50 Assessment

The correct dose to determine the mortality level for 50% of the population ($LD_{50}$) was assessed prior to the main challenge. A total of 220 fish (*Salmo salar*) from the same stock of fish used in the feeding experiment were distributed in 4 x 700L tanks. The fish had an average weight of 120 to 150 g (55 fish per tank). The tanks were supplied with sea water at room temperature. Once the fish were adjusted to the tank conditions they were injected (0.2 ml fish, intra-peritoneal injection, medial ventral) with 4 dilutions of a known titer of *P. salmonis* PSLT8 (1/10, 1/100, 1/1000 & 1/10,000). Water temperature and mortality data of injected fish were recorded over a 30 day-period. The estimate of the $LD_{50}$ was performed in parallel with the feeding period of the different diets.

Statistics

Mortalities were expressed as cumulative mortality (%). Data was assessed using a Kaplan-Meier survival analysis, in addition to a Log Rank & Wilcoxon test (Minitab 13.32, State College, PA, USA). Relative Percent Survival (RPS) was calculated as:

$$RPS = (1 - \frac{Mortality\, in\, Test\, group}{Mortality\, in\, Control\, group}) \times 100$$

Results and Discussion

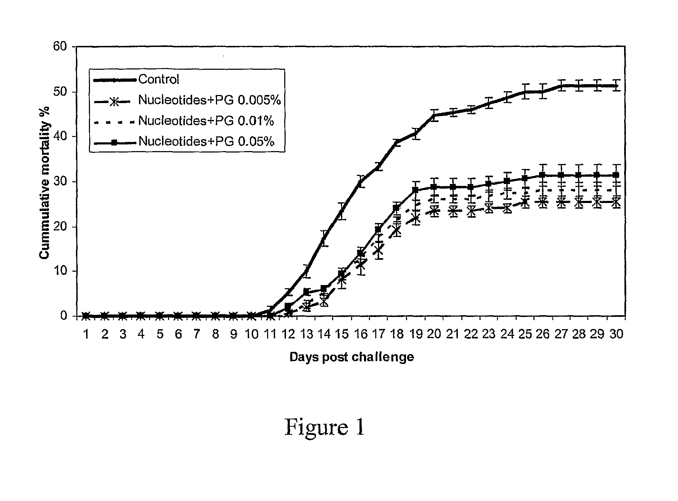

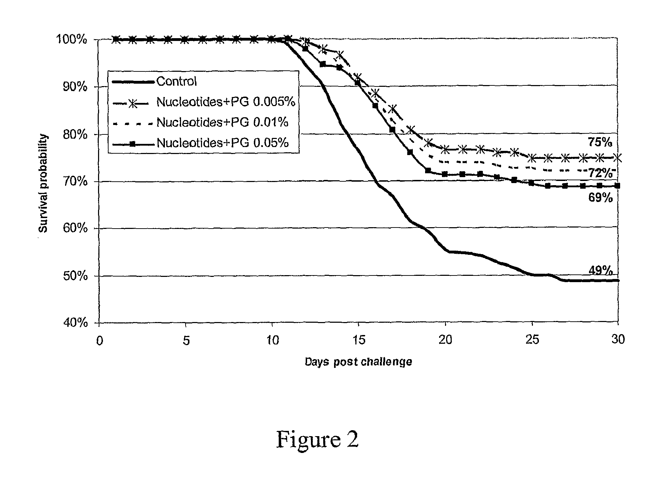

Fish fed nucleotides and peptidoglycan (PG) had a significantly higher Relative Percent Survival (RPS) by the end of the study after 30 days post-challenge (Table 4, Figure 2) (Log-Rank test: $p < 0.001$; Wilcoxon test: $p < 0.001$) compared to fish receiving the control diet. The probability of survival in the control group was below 49%, compared to 69%, 72% and 75% in the treatments receiving nucleotides and PG 0.05%, nucleotides and PG 0.01% and nucleotides and PG 0.005%, respectively (Figure 2). There were no significant differences in mortality between the treatments receiving diets comprising a combination of nucleotides and peptidoglycan, although a clear trend for lower mortalities with lower dose was observed (Figure 1). There was low deviation in mortalities between tanks and no significant difference of tank effect was observed (Log-Rank test: p=0.906; Wilcoxon test: p=0.952).

Table 4: Relative Percent Survival (RPS) and mortality percentage at the end of the post-challenge phase (mean ± standard error of the mean) of Atlantic salmon (*Salmo salar*) which were fed different diets prior to the *P. salmonis* challenge. * indicates significant differences (p<0.001 compared to the control.

| Treatment 1 | RPS (%) | Mortality (%) |
|---|---|---|
| 1 (control) | - | 51 ± 1.23 |
| 2 | 51 | 25 ± 1.33* |
| 3 | 46 | 28 ± 1.79* |
| 4 | 40 | 31 ± 2.40* |

Experiment 2

A total of 900 Atlantic salmon *Salmo salar* (150 fish per treatment, 6 treatments), start weight 80g, seawater adapted, were pit-tagged to identify the different groups, before the start of the feeding period. Fish were maintained in ambient seawater tempertature (13°C). Fish were fed for a period for 8 weeks at 2% body weight per day with six different diets in different combinations: a control diet without additional ingredients, a diet comprising nucelotides, a diet comprising peptidoglycan and diets comprising combinations of nuclotides and peptidoglycan (Table 5). Apart from the addition of nucleotides and/or peptidolgycan, all diets contained conventional feed ingredients (Table 6). In practice, the fish has free access to feed.

Table 5: Test diets used in experiment 2. PG = peptidoglycan.

| Diet No. | Diet name |
|---|---|
| 1. | Control diet (Commercial) |
| 2. | Diet with 0.2 % nucleotides |
| 3. | Diet with PG 0.005%; (50g PG tonne$^{-1}$) |
| 4. | Diet with 0.2 % nucleotides + PG 0.005% (50g PG tonne$^{-1}$) |
| 5. | Diet with 0.2 % nucleotides + PG 0.001% (10g PG tonne$^{-1}$) |

Table 6: Main feed ingredients and chemical analysis of the composition of the test diets in experiment 2.

| Ingredient (% by weight) | Diet 1 | Diet 2 | Diet 3 | Diet 4 | Diet 5 |
|---|---|---|---|---|---|
| Fish meal | 51 | 51 | 51 | 51 | 51 |
| Fish oil | 18 | 18 | 18 | 18 | 18 |
| Wheat gluten | 7 | 7 | 7 | 7 | 7 |
| Corn gluten meal | 8 | 8 | 8 | 8 | 8 |
| Vitamin premix* | 0.2 | 0.2 | 0.2 | 0.2 | 0.2 |
| Mineral premix** | 0.3 | 0.3 | 0.3 | 0.3 | 0.3 |
| Peptidoglycan | - | - | 0.005 | 0.005 | 0.001 |
| Nucleotide | - | 0.2 | - | 0.2 | 0.2 |
| **Composition** (%) | | | | | |
| Protein | 47.6 | 47.6 | 47.6 | 47.6 | 47.6 |
| Lipid | 24.3 | 24.3 | 24.3 | 24.3 | 24.3 |
| Ash | 7.5 | 7.5 | 7.5 | 7.5 | 7.5 |
| Dry mass | 92.7 | 92.5 | 92.7 | 92.5 | 92.7 |

*EWOS Vitamin premix ®
**EWOS Mineral premix ®.

Fish were fed the differed diets during the pre-challenge phase of 4 weeks (Table 7): Treatment 1 received a control diet without any addtional ingredients during the 4 weeks of the pre-challenge period. Treatment 2 received a diet with added nucleotides for 4 weeks. In treatment 3 fish were fed the controll diet for 3 weeks followed by a diet comprising 0.005 %PG for 1 week. In treatment 4 and 5 fish were fed the diet comprising nucleotides for 3 weeks followed by diets comprising to different combinations of nucleotides and peptidoglycan (0.005% PG in treatment 4 and 0.001 % PG in treatment 5) for 1 week. In treatment 6 fish were fed a diet comprising a combination of nucleotides and peptidoglycan for 4 weeks. After the pre-challenge period fish were challenged with *P. salmonis* as described in detail below.

During the post-challenge priod, all treatments were fed the control diet for 4 weeks until the experiment was finished.

Table 7. Feeding regime in experiment 2 before the fish were challenged with *Piscirikettsia salmonis* (pre-challenge) and after they were challenged with *P. salmonis* (post-challenge). Positive and negative controls are conventional commercial diets with added nucleotides (Diet 2) and without added nucleotides (Diet 1).

| Treatment | Pre-challenge | | | | Post-challenge | | | |
|---|---|---|---|---|---|---|---|---|
| | Week 1 | Week 2 | Week 3 | Week 4 | Week 5 | Week 6 | Week 7 | Week 8 |
| 1 | Diet 1 | Diet 1 | Diet 1 | Diet 1 | | | | |
| 2 | Diet 2 | Diet 2 | Diet 2 | Diet 2 | | Diet 1 | | |
| 3 | Diet 1 | Diet 1 | Diet 1 | Diet 3 | | | | |

(continued)

| Treatment | Pre-challenge | | | | Post-challenge | | | |
|---|---|---|---|---|---|---|---|---|
| | Week 1 | Week 2 | Week 3 | Week 4 | Week 5 | Week 6 | Week 7 | Week 8 |
| 4 | Diet 2 | Diet 2 | Diet 2 | Diet 4 | | | | |
| 5 | Diet 2 | Diet 2 | Diet 2 | Diet 5 | | | | |
| 6 | Diet 5 | Diet 5 | Diet 5 | Diet 5 | | | | |

Challenge with *Piscirikettsia salmonis*

After four weeks feeding test diets (pre-challende period), the fish were distributed equally into 6 tanks with 25 fish per group per tank (150 fish total per tank). Fish were challenged with 0.2 ml of a dose predicted to kill 50% of the population ($LD_{50}$) of *Piscirikettsia salmonis* by intra-peritoneal (i.p.) injection. Fish were fed control diets throughout the challenge period. Dead fish were removed daily and recorded.

$LD_{50}$ Assessment:

The estimate of the $LD_{50}$ was performed as described for experiment 1 in parallel with the feeding period of the different diets. The average weight of fish for the $LD_{50}$ assessment was 80 g (55 fish per tank).

Statistics

Statictical assessments were carried out as described in experiment 1.

Results and Discussion

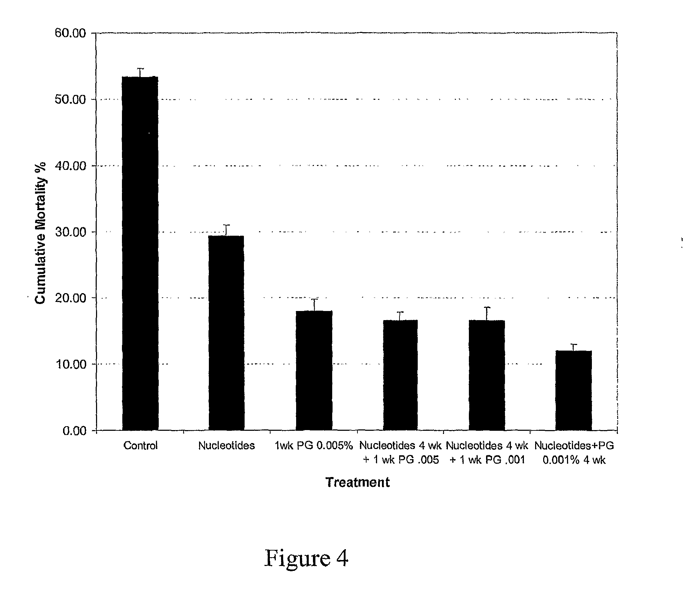

The $LD_{50}$-dose was administered to achieve a level of mortality in the control population of approximately 50%. The final control mortality reached 53% (Table 8, figure 4). There appeared to be no significant tank-based effect in the current challenge study (p=0.967).

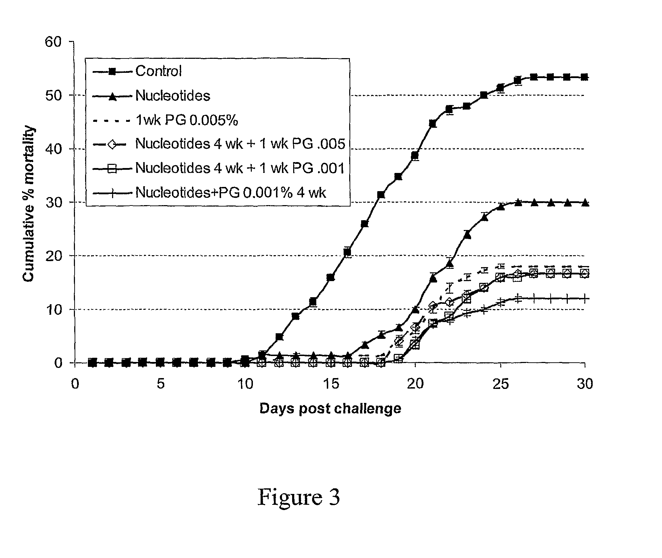

At 14 days post-challenge mortalities had passed 10% in the control treatment (Figure 3). In the groups fed peptidoglycan mortalities reached 10% after 21 days post challenge. Nucleotide addition alone also delayed, as well as reduced the overall mortality (Figure 4), however not as efficient as it was the case for the combination of peptidoglycan and nucleotides. Fish receiving the diet only comprising nucleotides as an additive showed a Relative Percent Survival (RPS) of 45% (Table 8) and a significantly higher survival probability (p<0.001, Log Rank-Wilcoxon) compared to the control receiving the control diet without any additive.

All treatments receiving peptidoglycan had significantly higher survival probabilities (p>0.001, Log Rank-Wilcoxon) with RPS values ranging from 66% to 78% compared to the treatments receiving the control diet. (Table 8, Figure 3).

Table 8. Percentage mortality and Relative Percent Survival (RPS) of Atlantic salmon (*Salmo salar*) fed different diets prior to the *Piscirickettsia salmonis* challenge. SEM= Standard error of means.

| Treatment | Average mortality (%) | SEM | RPS |
|---|---|---|---|
| 1 (control) | 53.33 | 1.33 | - |
| 2 | 29.33 | 1.69 | 45 |
| 3 | 18.00 | 1.71 | 66 |
| 4 | 16.67 | 1.23 | 69 |
| 5 | 16.67 | 1.91 | 69 |
| 6 | 12.00 | 1.03 | 78 |

The peptidoglycan groups all had significantly different survival probabilities from the treatment receiving only nucleotides as an additive (p<0.05, Log Rank-Wilcoxon).

Fish fed diets comprising a combination of nucleotides and peptidoglycan (treatment 4, 5 and 6) in the pre-challenge phase had 3-12% higher survival probabilities compared to fish fed a diet only comprising peptidoglycan (treatment 3) without any addition of nucleotides.

There were no differences in survival in fish fed a diet with nucleotides for 3 weeks followed by a diet comprising nucleotides in combination with different amounts of peptidoglycan (0.001% PG or 0.005% PG). However, best post-challenge survival was surprisingly achieved when fish were fed a combination of peptidoglycan in a low concentration (0.001% PG) in combination with nucleotides for the whole pre-challenge period of 4 weeks (treatment 6).

Conclusions from experiment 1 and 2:

Feeding a combination of nucleotides and peptidoglycan in experiment 1 significantly increased the resistance to *Piscirickettsia salmonis,* compared to the negative control (Commercial diet). There was no significant difference in survival between test groups (peptidoglycan + nucleotides) although there was a trend for increased survival with lower dose in experiment 1.

Zhou et al. (2006; "Effects of dietary supplementation of A3$\alpha$-peptidoglycan on innate immune responses and defense activity of Japanese flounder (Paralichthys olivaceus)", Aquaculture 251, 172-181) found 4g kg$^{-1}$ diet as the optimal dose of peptidoglycan in Japanese flounder (*Paralichthys olivaceus*), following challenge with *Vibrio anguillarium.* Doses both lower and higher than 4g kg$^{-1}$ are reported to increase mortalities. Similar dose ranging effects were observed with black tiger prawns *(Penaeus monodon)* where improved survival was achieved at a lower optimal dose (Boonyaratpalin S. et al. (1995) "Effects of peptidoglycan prepared from Brevibacterium lactofermentum on growth, survival, immune response, and tolerance to stress in black tiger shrimp, Penaeus monodon." In: Disease in Asian Aquaculture II. M. Shariff, J.R. Arthur & R.P. Subasinghe (eds.), p. 469-477. Fish Health Section, Asian Fisheries Society, Manila, Philippines).

Surprisingly, we could show that a concentration of 0.5g-0.01g PG kg$^{-1}$ diet in combination with nucleotides was effective to significantly improve survival of infected fish in experiment 1 and 2. Moreover, peptidoglycan was effective in a concentration as low as 0.05g kg$^{-1}$ feed in improvement of post-challenge survival in experiment 2 without an addition of nucleotides.

Oral administration of peptidoglycan in combination with nucleotides during the pre-challenge period in experiment 2 gave significant protection in survival of fish at the end of the post-challenge period of up to 78% RPS (survival probability p<0.001, Log Rank-Wilcoxon).

Best results were achieved when peptidoglycan was administered to the fish in a low concentration (0.001% PG; final dose 6 mg kg$^{-1}$ body weight) in combination with nucleotides during the whole pre-challenge phase.

The results of the experiments suggest that adequate levels of nucleotides administered along with the immune stimulant are probably beneficial for the fish in periods of challenges or stress by infections. These effects may be due to nucleotides becoming limiting when cell populations are rapidly increased e.g. during periods of immune system stimulation.

Negative effects on immunity due to over-exposure to peptidoglycan have been reported in the literature (Matsuo & Miyazono, (1993) reported that oral administration of PG at 0.2 and 2 mg/kg diet enhances the disease resistance of rainbow trout against *V. anguillarum* infection but a longer administration than 28 days can lead to decrease in disease resistance.

The results of the experiments suggest that nucleotides may compensate for the these negative effects related to an over-exposure and/or long-term by peptidoglycan.

The present invention thus represents an important improvement for the health management of fish.

It will be appreciated that the features of the invention described in the foregoing can be modified without departing from the scope of the invention.

Definitions of terms:

The term *peptidoglycan* comprises all commonly described and not yet described compounds belonging to the substance group of peptidoglycans.

*Nucleotides* comprise any known phosphor ester of a nucleoside such as AMP, GMP, UMP, CMP, UMP.

*Conventional feed ingredients* are feed ingredients which are commonly used in feed compositions for a specific animal species such as lipids, proteins, vitamins, carbohydrates, minerals, etc.

A *functional feed* which can be similar in appearance to, or may be, a conventional food that is consumed as part of a usual diet, and is demonstrated to have physiological benefits and/or reduce the risk of certain diseases beyond basic nutritional functions, i.e. by comprising bioactive compounds such as nucleotides and peptidoglycan.

*Salmonids* are fish belonging to the family of Salmonidae. Representative examples are Atlantic salmon (*Salmo salar*), Rainbow trout *(Onchorynchus mykiss),* Coho salmon *(Onchorynchus kisutch).*

The term *infectious disease* includes commonly known infectious diseases of animals e. g. caused by bacteria such as *Piscirikettsia salmonis, Moritella viscosa, Francisella* sp, Mouth Rot, Streptococcal infections, *Vibrio* infections; Pancreas disease, NSAV, gill inflammation (GI), heart and skeletal muscle inflammation (HSMI), infectious salmonid anaemia (ISA) virus, Saprolegniosis, and sealice infestation.

The term *recovery* means that the health of an animal is restored after it has been affected by a disease.

**Claims**

1. Fish feed composition comprising conventional feed ingredients **characterized in that** said feed composition comprises peptidoglycan and nucleotides, wherein said peptidoglycan is in the range of 0.001-0.01%, more preferable 0.001-0.005 % and most preferably 0.005% by weight.

2. Feed composition according to claim 1, **characterized in that** the nucleotides are in the range of 0.05 % -1 % by weight, preferably 0.1-0.5 % by weight and most preferably of 0.2 % by weight.

3. Feed composition according to claim 1, **characterized in that** the weight ratio of nucleotides to peptidoglycan is more than 4, more preferable more than 20, and more preferable more than 40, and the amount of peptidoglycan is in the range of 0.001-0.01 % of the weight of the feed composition.

4. Feed composition according to claim 1, wherein the amount of nucleotides is about 0.2% and the amount of peptidoglycan is about 0.005% of the total weight of the feed composition.

5. Feed composition comprising conventional feed ingredients, nucleotides, and peptidoglycan for use in the prophylaxis and/or treatment of infectious diseases in fish, and/or for use in the reduction of symptoms of an infectious disease, wherein the peptidoglycan is in the range of 0.001-0.01%.

6. Feed composition according to claim 5, wherein said peptidoglycan is in the range of 0.001-0.005 % and preferably 0.005% by weight.

7. Feed composition according to claim 5, wherein said composition is provided to the fish in the period previous of the challenge by an infection, during the infection or after the fish has been infected.

8. Feed composition according to claim 5, wherein the weight ratio of nucleotides to peptidoglycan is more than 4, more preferable more than 20, and more preferable more than 40.

9. Feed composition according to any of the claims 5-8 as a functional feed composition to prevent or reduce the symptoms related of an infectious disease in a fish.

10. Feed composition according to any of the claims 5-9 to increase survival and/or growth in fish challenged by an infection.

11. Feed composition according to any of the claims 5 to 10, where the infection is caused by bacteria such as *Piscirikettsia salmonis, Moritella viscosa, Francisella* sp, Mouth Rot, Streptococcal infections, *Vibrio* infections; Pancreas disease, NSAV, gill inflammation (GI), heart and skeletal muscle inflammation (HSMI), infectious salmonid anaemia (ISA) virus, Saprolegniosis, and sea lice infestation.

12. Feed composition according to any of the claims 5 to 11, wherein the fish is a salmonid, preferable an Atlantic salmon..

13. Feed composition according to claim 5, for effective recovery following an infection and/or antibiotic treatment due to an infection and/or to reduce the risk of a re-infection.

14. Composition according to any of the claims 1-4 for use in feeding of fish which are susceptible to an infection wherein the composition is provided to the fish in the period previous of the challenge by an infection, wherein the composition is fed for a period of 1-12 weeks prior to the infection.

15. Composition for use according to claim 14, wherein the composition is fed for a period of 2-6 weeks prior to infection, and most preferably of 4 weeks prior to infection.

16. Composition for use according to claim 14, wherein the fish is a salmonid, preferable an Atlantic salmon.

**Patentansprüche**

1. Fischfutterzusammensetzung, die übliche Futterbestandteile umfasst, **dadurch gekennzeichnet, dass** die Futterzusammensetzung Peptidoglykan und Nukleotide umfasst, wobei das Peptidoglykan im Bereich von 0,001-0,01 Gew.-% liegt, bevorzugt im Bereich von 0,001-0,005 Gew.-% und besonders bevorzugt im Bereich von 0,005 Gew.-%.

2. Futterzusammensetzung nach Anspruch 1, **dadurch gekennzeichnet, dass** die Nukleotide im Bereich von 0,05 Gew.-% - 1 Gew.-% liegen, vorzugsweise im Bereich von 0,1-0,5 Gew.-% und besonders bevorzugt im Bereich von 0,2 Gew.-%.

3. Futterzusammensetzung nach Anspruch 1, **dadurch gekennzeichnet, dass** das Gewichtsverhältnis von Nukleotiden zu Peptidoglykan mehr als 4 beträgt, bevorzugt mehr als 20, und besonders bevorzugt mehr als 40, und dass die Menge an Peptidoglykan im Bereich von 0,001-0,01% des Gewichts der Futterzusammensetzung liegt.

4. Futterzusammensetzung nach Anspruch 1, wobei die Menge an Nukleotiden etwa 0,2% und die Menge an Peptidoglykan etwa 0,005% des Gesamtgewichts der Futterzusammensetzung beträgt.

5. Futterzusammensetzung, die übliche Futterbestandteile, Nukleotide und Peptidoglykan zur Verwendung bei der Prophylaxe und/oder Behandlung von Infektionskrankheiten beim Fisch und/oder zur Verwendung bei der Verringerung von Symptomen einer Infektionskrankheit umfasst, wobei das Peptidoglykan im Bereich von 0,001-0,01% liegt.

6. Futterzusammensetzung nach Anspruch 5, wobei das Peptidoglykan im Bereich von 0,001-0,005 Gew.-% und vorzugsweise im Bereich von 0,005 Gew.-% liegt.

7. Futterzusammensetzung nach Anspruch 5, wobei die Zusammensetzung dem Fisch in dem Zeitraum vor der Belastung durch eine Infektion, während der Infektion oder nachdem der Fisch infiziert wurde verabreicht wird.

8. Futterzusammensetzung nach Anspruch 5, wobei das Gewichtsverhältnis von Nukleotiden zu Peptidoglykan mehr als 4, bevorzugt mehr als 20 und besonders bevorzugt mehr als 40 beträgt.

9. Futterzusammensetzung nach einem der Ansprüche 5-8 als zweckmäßige Futterzusammensetzung zum Verhindern oder Verringern der mit einer Infektionskrankheit bei einem Fisch verbundenen Symptome.

10. Futterzusammensetzung nach einem der Ansprüche 5-9 zur Erhöhung des Überlebens und/oder Wachsens bei durch eine Infektion belastetem Fisch.

11. Futterzusammensetzung nach einem der Ansprüche 5 bis 10, wobei die Infektion durch Bakterien wie Piscirikettsia salmonis, Moritella viscosa, Francisella sp, Mundfäule, Streptokokken-Infektionen, Vibrio-Infektionen; Pankreaserkrankung, NSAV (Norwegian Salmonid Alphavirus), Kiemenentzündung (GI), Herz- und Skelettmuskel-Entzündung (HSMI), Virus der infektiösen Lachsanämie (ISA), Saprolegniose und Seelausbefall verursacht wird.

12. Futterzusammensetzung nach einem der Ansprüche 5 bis 11, wobei der Fisch ein Lachsfisch, vorzugsweise ein Atlantischer Lachs, ist.

13. Futterzusammensetzung nach Anspruch 5 für eine erfolgreiche Gesundung nach einer Infektion und/oder Behandlung mit Antibiotika aufgrund einer Infektion und/oder zur Verringerung der Gefahr einer erneuten Infektion.

14. Zusammensetzung nach einem der Ansprüche 1-4 zur Verwendung beim Füttern von infektionsanfälligem Fisch, wobei die Zusammensetzung dem Fisch in dem Zeitraum vor der Belastung durch eine Infektion verabreicht wird, wobei die Zusammensetzung über einen Zeitraum von 1-12 Wochen vor der Infektion gefüttert wird.

15. Zusammensetzung zur Verwendung nach Anspruch 14, wobei die Zusammensetzung über einen Zeitraum von 2-6 Wochen, und besonders bevorzugt über einen Zeitraum von 4 Wochen, vor der Infektion gefüttert wird.

16. Zusammensetzung zur Verwendung nach Anspruch 14, wobei der Fisch ein Lachsfisch ist, vorzugsweise ein Atlantischer Lachs.

**Revendications**

1. Composition alimentaire pour poissons comprenant des ingrédients alimentaires classiques **caractérisée en ce que** ladite composition alimentaire comprend un peptidoglycane et des nucléotides, ledit peptidoglycane étant présent dans la plage de 0,001 à 0,01 %, de préférence 0,001 à 0,005 %, et mieux encore à raison de 0,005 % en poids.

2. Composition alimentaire selon la revendication 1, **caractérisée en ce que** les nucléotides sont présents dans la plage de 0,05 à 1 % en poids, de préférence 0,1 à 0,5 % en poids et mieux encore à raison de 0,2 % en poids.

3. Composition alimentaire selon la revendication 1, **caractérisée en ce que** le rapport en poids des nucléotides au peptidoglycane est supérieur à 4, de préférence supérieur à 20, et mieux encore supérieure à 40, et la quantité de peptidoglycane est dans la plage de 0,001 à 0,01 % en poids de la composition alimentaire.

4. Composition alimentaire selon la revendication 1, dans laquelle la quantité de nucléotides est d'environ 0,2 % et la quantité de peptidoglycane est d'environ 0,005 % du poids total de la composition alimentaire.

5. Composition alimentaire comprenant des ingrédients alimentaires classiques, des nucléotides, et un peptidoglycane destinée à être utilisée pour prévenir et/ou traiter des maladies infectieuses chez le poisson, et/ou destinée à être utilisée pour réduire les symptômes d'une maladie infectieuse, le peptidoglycane étant présent dans la plage de 0,001 à 0,01 %.

6. Composition alimentaire selon la revendication 5, dans laquelle ledit peptidoglycane est présent dans la plage de 0,001 à 0,005 % et de préférence à raison de 0,005 % en poids.

7. Composition alimentaire selon la revendication 5, dans laquelle ladite composition est administrée au poisson dans la période précédant la provocation par une infection, pendant l'infection ou après que le poisson a été infecté.

8. Composition alimentaire selon la revendication 5, dans laquelle le rapport en poids des nucléotides au peptidoglycane est supérieur à 4, de préférence supérieur à 20, et mieux encore supérieur à 40.

9. Composition alimentaire selon l'une quelconque des revendications 5 à 8 à titre de composition alimentaire fonctionnelle destinée à prévenir ou à réduire les symptômes liés à une maladie infectieuse chez un poisson.

10. Composition alimentaire selon l'une quelconque des revendications 5 à 9 destinée à accroître la survie et/ou la croissance chez un poisson provoqué par une infection.

11. Composition alimentaire selon l'une quelconque des revendications 5 à 10, dans laquelle l'infection est provoquée par des bactéries telles que *Piscirikettsia salmonis, Moritella viscosa, Francisella* sp., la pourriture de la bouche, les infections à streptocoques, les infections à *Vibrio ;* une maladie du pancréas, l'alpha-virus des salmonidés norvégiens (NSAV), l'inflammation des ouïes (GI), l'inflammation du muscle cardiaque et squelettique (HSMI), le virus de l'anémie infectieuse des salmonidés (ISA), la saprolégniose, et l'infestation par des limaces de mer.

12. Composition alimentaire selon l'une quelconque des revendications 5 à 11, dans laquelle le poisson est un salmonidé, de préférence un saumon de l'Atlantique.

13. Composition alimentaire selon la revendication 5, pour un rétablissement efficace après une infection et/ou un traitement aux antibiotiques consécutif à une infection, et/ou la réduction du risque de réinfection.

14. Composition selon l'une quelconque des revendications 1 à 4 destinée à être utilisée pour nourrir des poissons qui sont sensibles à l'infection dans laquelle la composition est administrée aux poissons dans la période précédant la provocation par une infection, la composition étant administrée pendant une période de 1 à 12 semaines avant l'infection.

15. Composition destinée à être utilisée selon la revendication 14, dans laquelle la composition est administrée pendant une période de 2 à 6 semaines avant l'infection, et mieux encore de 4 semaines avant l'infection.

16. Composition destinée à être utilisée selon la revendication 14, dans laquelle le poisson est un salmonidé, de préférence un saumon de l'Atlantique.

Figure 1

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1082908 A1
- WO 2007095313 A2
- WO 2008051091 A1

### Non-patent literature cited in the description

- **MATSUO K. ; MIYAZONO I.** The influence of long-term administration of peptidoglycan on disease resistance and growth of juvenile Rainbow-trout. *Nippon Suisan Gakkaishi,* August 1993, vol. 89 (8), 1377-1379
- **ZHOU et al.** Effects of dietary supplementation of A3α-peptidoglycan on innate immune responses and defense Japanese flounder (Paralichthys olivaceus). *Aquaculture,* 2006, vol. 251, 172-181
- **BURRELLS et al.** Dietary nucleotides: a novel supplement in fish feeds. Effects on resistance to disease in salmonids. *Aquaculture,* 2001, vol. 199, 159-169
- **LI et al.** Nucleotide nutrition in fish: Current knowledge and future applications. *Aquaculture,* 2006, vol. 251, 141-152
- **ZHOU et al.** Effects of dietary supplementation of A3α-peptidoglycan on innate immune responses and defense activity of Japanese flounder (Paralichthys olivaceus). *Aquaculture,* 2006, vol. 251, 172-181
- Effects of peptidoglycan prepared from Brevibacterium lactofermentum on growth, survival, immune response, and tolerance to stress in black tiger shrimp, Penaeus monodon. **BOONYARATPALIN S. et al.** Disease in Asian Aquaculture II. Asian Fisheries Society, 1995, 469-477